# EUROPEAN PATENT APPLICATION

(11) **EP 3 881 864 A1**
(43) Date of publication of application: **22.09.2021**
(21) Application number: 20164690.8
(22) Date of filing: 20.03.2020
(51) Int. Cl.: A61K 39/015, C07K 16/20, A61K 39/00

(54) **VACCINES INDUCING IMPROVED GLYCOSYLATION OF IGG ANTIBODIES**

(71) Applicant: Sanquin Innovatie B.V., 1066 CX Amsterdam (NL)
(72) Inventor: VIDARSSON, Gestur, 1066 CX Amsterdam (NL)
(74) Representative: V.O.

(57) **Abstract**

The invention relates to mammalian cell or a cell membrane containing fraction thereof comprising a viral, bacterial, parasitic or fungal antigen at its cell surface, to live attenuated enveloped virus comprising a *Plasmodium* antigen or a nucleic acid sequence encoding said *Plasmodium* antigen or comprising a tumor-associated antigen or a nucleic acid sequence encoding said antigen, compositions and vaccines comprising these and uses thereof.

## Description

### Field of the invention

The present invention relates to the field of vaccination, more in particular to vaccination against microorganisms or tumor antigens.

### Background of the invention

Antibodies are antigen recognizing immunoglobulins with an diverse repertoire in the antigen specific domain. Modifications such as somatic recombination and hyper mutation increases the diversity of antibodies while variation in the isotype and post-translational modifications such as Fc glycosylation further increase diversity of their effector functions. Posttranslational modification of proteins by glycosylation is well known in the art and most membrane surface proteins and secreted proteins found in plasma are glycosylated, including antibodies. The presence of a conserved N-linked glycan at position 297, in the so called constant Fc-domain of IgG is essential for effector functions. However, it is now generally accepted that the composition of this glycan is highly variable and has functional consequences. This is especially true for the core fucose moiety in the Fc glycan.

Fc-mediated immune effector function, antibody-dependent cellular cytotoxicity (ADCC) and phagocytosis, are two major modes of action of antibodies resulting in depletion of cells whose membrane-surface antigens have been bound by specific antibodies. This effector function is modulated by the N-linked glycosylation in the Fc region of the antibody. Interestingly, removal of the core fucose on the Fc N-glycan of IgG1 antibodies increases IgG1 Fc binding affinity to the FcγRIIIa receptors present on immune effector cells such as natural killer cells, leading to enhanced ADCC activity. Similarly, afucosylated antibodies also bind stronger to FcγRIIIb on granulocytes resulting in elevated phagocytosis. The discovery that IgG variants without core-fucosylation causes elevated antibody dependent cellular cytotoxicity (ADCC), via increased IgG-Fc-receptor IIIa (FcγRIIIa) affinity, resulted in altered formulation of several next-generation glyco-engineered monoclonal antibody (mAb) therapies without core-fucosylation for targeting tumors and autoimmune disorders. The current strategies are mainly based on recombinant production of engineered afucosylated antibodies which can subsequently be administered to patients. Afucosylated antibodies can be produced by for example, engineering the Fc region through amino acid mutations and/or glycoengineering the Fc N-glycan to the reduce core fucose. In humans, changes in the Fc glycans are associated with age, sex and autoimmune diseases. However, total serum IgG antibodies are exclusively fucosylated at birth and slightly decrease to ∼94% fucosylation at adulthood. Until now, no strong clues on how or if IgG core fucosylation is controlled have come forward.

IgG antibodies are crucial for protection against invading pathogens and are the most abundant class of antibodies found in human plasma. There are four subclasses (IgG1, IgG2, IgG3, and IgG4), each different in the structure of their constant regions (Fc domain). Further, each subclass can trigger antibody-dependent cellular cytotoxicity (ADCC) and complement-dependent cytotoxicity (CDC). IgG is composed of three globular domain structures, two of which are the fragments for antigen binding (Fab) and the other is the fragment crystallizable (Fc) that activates Fcγ receptors (FcyRs) on leukocytes and C1 component of complement. IgG molecules bear oligosaccharides at Asn297 of the Fc region, and the oligosaccharide plays an essential role in Fc effector functions including ADCC and CDC. The Fc region acts as an important bridge between adaptive and innate immune response. When antigens expressed on the surfaces of cancer cells, microorganism infected cells or invading pathogens are recognized by specific antibodies, the cells or pathogens become bound by antibodies. The Fc region of the antibodies assists in the elimination of the targets via different mechanisms. For example, the Fc region can interact with the C1 molecule of the complement system and trigger the activation of classical pathway of the complement system. It can also recruit phagocytes via Fc receptors and activate the phagocytosis pathway and/or ADCC.

Vaccination is a robust method to induce defense against pathogens, as have been demonstrated by many serious viral diseases such as smallpox and poliomyelitis (polio) which have been nearly eradicated. There are three major types of vaccines: subunit vaccines, inactivated (killed) pathogen vaccines, and attenuated live pathogen vaccines. Subunit vaccines are based on components, proteins or antigens of the pathogen that best stimulate the immune system. Their efficacy, however, can be low because the antigens alone are not always sufficient to induce adequate long-term immunity. Thus, it often requires the incorporation of adjuvants to elicit a stronger protective immune response.

Inactivated (killed) pathogen vaccines are made by growing the (wildtype) pathogen in culture followed by inactivation of the pathogen, for example, with formaldehyde. A great deal of experimentation is required to find an inactivation treatment that efficiently kills all of the pathogen without destroying the immunogenic particles. Such vaccines can elicit strong protective immune responses. Hence, many of the vaccines used in clinic fall into this category. However, not every infectious diseases can be effectively prevented with an inactivated pathogen vaccine. In addition, residual safety issues concerns remain for growing certain pathogens e.g. in case of failure of the inactivation.

An attenuated live pathogen vaccine comprises live, whole pathogens that are treated in such a way that they have reduced virulence within the host but retain their ability to provoke an immune response. For example, by introducing mutations or by growing the pathogen in a series of tissue culture steps followed by selection for the less virulent strains. Thus, the pathogen is kept alive and may still be able to replicate. Live attenuated pathogen vaccines offer considerable advantages over inactivated (killed) vaccines as they activate a wider range of immune responses, induce long-lasting and rapid onset of immunity, often reduce the need for booster vaccinations and adjuvants, can be produced at relatively low cost, and live attenuated viruses can be even administered by the oral route. These advantages are particularly important in an emergency, when a vaccine is rapidly needed.

Despite the current advances in vaccine development, many viruses such as rhinovirus and influenza virus are still poorly controlled, and these viruses still create substantial problems, though these problems vary from year to year and country to country. In addition, new virus strains, such as Human Immunodeficiency Virus (HIV), Influenza virus and Corona virus, regularly appear in human populations and often cause deadly pandemics. The current vaccination approaches have also proven ineffective for vaccines directed against metazoan parasites or human parasitic diseases such as malaria. About 216 million cases of malaria are reported each year with the most virulent *Plasmodium* species being *Plasmodium falciparum* (*P. falciparum*)*,* which accounts for the vast majority of deaths. Immunization of large numbers of individuals using sporozoites remains challenging and the development of a subunit vaccine (that contains only the antigenic parts of a pathogen) has not succeeded despite decades of research. Also vaccinations against parasite resulting in high antibody titers can still provide insufficient protection. One particular example is MSP-1 malaria vaccine, which generated high titers of antibodies but still failed to protect against infection.

Thus, there remains a need to improve current and future vaccination strategies to induce a stronger, broader and/or long-lasting immunity.

### Summary of the invention

It is an object of the invention to provide improved vaccination strategies, for instance by inducing an immune response in a subject that provides a stronger protection than current vaccination methods. It is a further object to provide a solution for the challenges in the development of vaccines against "difficult" targets.

The invention therefore provides a mammalian cell or a cell membrane containing fraction thereof comprising a viral, bacterial, parasitic or fungal antigen at its cell surface.

In a further aspect, the invention provides a live attenuated enveloped virus comprising a *Plasmodium* antigen or a nucleic acid sequence encoding said *Plasmodium* antigen.

In a further aspect, the invention provides a live attenuated enveloped virus comprising a tumor-associated antigen or a nucleic acid sequence encoding said antigen.

In a further aspect, the invention provides a nucleic acid construct comprising a nucleic acid sequence encoding a live attenuated enveloped virus according to the invention.

In a further aspect, the invention provides a pharmaceutical composition comprising a cell or fraction thereof or a live attenuated enveloped virus according to the invention and a pharmaceutically acceptable carrier, diluent and/or excipient.

In a further aspect, the invention provides a vaccine comprising a cell or fraction thereof or a live attenuated enveloped virus according to the invention and a pharmaceutically acceptable carrier, diluent and/or excipient.

In a further aspect, the invention provides a cell or fraction thereof or a live attenuated enveloped virus according to the invention for use as a vaccine.

In a further aspect, the invention provides a method of vaccination comprising administering to the subject a therapeutically effective amount of a cell or fraction thereof or a live attenuated enveloped virus according to the invention.

In a further aspect, the invention provides a use of a cell or fraction thereof or a live attenuated enveloped virus according to the invention in the manufacture of a medicament or vaccine.

In a further aspect, the invention provides a cell or fraction thereof or a live attenuated enveloped virus according to the invention for use in the treatment or prevention of a viral, bacterial, parasitic or fungal infection.

In a further aspect, the invention provides a method for treatment or prevention of a viral, bacterial, parasitic or fungal infection comprising administering to a subject a therapeutically effective amount of a cell or fraction thereof or a live attenuated enveloped virus according to the invention.

In a further aspect, the invention provides a use of a cell or fraction thereof or a live attenuated enveloped virus according to the invention in the manufacture of a medicament or vaccine for the treatment or prevention of a viral, bacterial, parasitic or fungal infection.

In a further aspect, the invention provides a cell or fraction thereof or a live attenuated enveloped virus according to the invention for use in the treatment or prevention of malaria.

In a further aspect, the invention provides a method for treatment or prevention of malaria comprising administering to a subject a therapeutically effective amount of a cell or fraction thereof or a live attenuated enveloped virus according to the invention.

In a further aspect, the invention provides a use of a cell or fraction thereof or a live attenuated enveloped virus according to the invention in the manufacture of a medicament for the treatment or prevention of malaria

In a further aspect, the invention provides a live attenuated enveloped virus comprising a tumor-associated antigen or a nucleic acid sequence encoding said antigen according to the invention for use in the treatment or prevention of cancer.

In a further aspect, the invention provides a method for treatment or prevention of cancer comprising administering to a subject a therapeutically effective amount of a live attenuated enveloped virus comprising a tumor-associated antigen or a nucleic acid sequence encoding said antigen according to the invention.

In a further aspect, the invention provides a use of a live attenuated enveloped virus comprising a tumor-associated antigen or a nucleic acid sequence encoding said antigen according to the invention for the manufacture of a medicament for the treatment or prevention of cancer.

In a further aspect, the invention provides a cell or fraction thereof or a live attenuated enveloped virus according to the invention for use in inducing and/or stimulating an afucosylated IgG response against an antigen of interest in a subject, wherein said cell or fraction thereof comprises said antigen of interest at its cell surface, or said virus comprises said antigen of interest or a nucleic acid encoding said antigen of interest, preferably wherein said afucosylated IgG molecules have increased binding to the FcγRIIIa and/or FcγRIIIb receptor.

In a further aspect, the invention provides a method for inducing and/or stimulating an afucosylated IgG response against an antigen of interest in a subject, wherein said cell or fraction thereof comprises said antigen of interest at its cell surface, or said virus comprises said antigen of interest or a nucleic acid encoding said antigen of interest, preferably wherein said afucosylated IgG molecules have increased binding to the FcyRIIIa and/or FcyRIIIb receptor, said method comprising administering to a subject in need thereof a cell or fraction thereof or a live attenuated enveloped virus according to the invention.

In a further aspect, the invention provides a cell or fraction thereof or a live attenuated enveloped virus according to the invention for use in inducing an immunogenic response against an antigen of interest in a subject, wherein said cell or fraction thereof comprises said antigen of interest at its cell surface, or said virus comprises said antigen of interest or a nucleic acid encoding said antigen of interest.

In a further aspect, the invention provides a method for inducing an immunogenic response against an antigen of interest in a subject, wherein said cell or fraction thereof comprises said antigen of interest at its cell surface, or said virus comprises said antigen of interest or a nucleic acid encoding said antigen of interest, said method comprising administering to a subject in need thereof a cell or fraction thereof or a live attenuated enveloped virus according to the invention.

### Detailed description

The inventors have now identified how an afucosylated immune response can be induced directly in a subject upon vaccination. This new strategy is based on the discovery that an increase in the afucosylated immune response is observed against foreign proteins found in the cell membrane of host cells.

As shown in the examples, the inventors discovered that antigen-specific antibodies against allo-antigen human platelet antigen (HPA)-1a show a strong decrease in fucosylation. Alloantibodies against red blood cells and platelets often show IgG Fc-fucosylation as low as 10% in most patients while the overall IgG-FC fucosylation is constantly high (∼94%) (R. Kapur, et al., A prominent lack of IgG1-Fc fucosylation of platelet alloantibodies in pregnancy. Blood 123, 471-481 (2014)). The low fucosylation results in an enhanced pathogenicity, suggesting an enhanced effector function of the antibodies.

Similar to antibodies against allo-antigens, the inventors found that immune responses to enveloped viruses show a significant decrease in fucosylation of antigen-specific IgG, while no decrease in fucosylation of IgG antibodies against non-enveloped viruses was observed, such as Parvo B19. Further, a lower level of IgG Fc-fucosylation was also found for natural occurring antibodies against enveloped viruses HIV and dengue but not for antibodies against inactivated vaccines, e.g. inactivated influenza, pneumococcal, meningococcal or tetanus vaccines. In the case of HIV, the low level of Fc-fucosylation has been associated with improved antiviral activity and HIV control.

Additionally, lower levels of IgG fucosylation of antibodies were observed in patients naturally infected with the enveloped virus Hepatitis B when compared to those vaccinated with recombinant Hepatitis B surface antigen. Interestingly, the IgG fucosylation levels of individuals naturally infected with Measles virus (MeV) and Mumps virus (MuV) was comparable to individuals that were vaccinated with live attenuated viruses for MeV and MuV.

Finally, in support of our hypothesis and importance of fucosylation for anti-malaria responses and future vaccine development, is the fact that current experimental malaria vaccins are recombinant soluble proteins (e.g. VAR-type malaria pathogenic proteins expressed on RBC), and were shown not to induce antibodies with afucosylated signatures. Thus these antibody bind with low affinity to FcγRIIIa and FcγRIIIb, and induce lower ADCC and phagocytosis.

These findings provide solid evidence that antibody fucosylation can be influenced by the context in which the antigen is presented to the immune system. Without wishing to be bound by theory, it is hypothesized that foreign membrane antigens that reside in a host membrane, such as surface membrane proteins in enveloped viruses, intracellular parasite or allogenic transplants, can specifically trigger an afucosylated antibody response. Thus, an afucosylated immune response in a subject is induced by the combined recognition of the pathogenic antigen and a self antigen by the host immune cells ("recognition of self hypothesis"). In this context the membrane of (infected) host cells express both exogenous and endogenous antigens providing a platform for co-recognition by host receptors on immune cells. This means that in order to gain strong antibody responses with high affinity for FcγRIIIa and FcγRIIIb, and thus a strong potential to induce ADCC and phagocytosis, immune response can be tailored by providing a vaccine in the right context, e.g. by using attenuated enveloped viruses or another approach whereby the antigen of interest is presented together with a self antigen of the subject.

Development of vaccines that induce strong and potent afucosylated immune responses by allowing recognition of the pathogenic antigen in combination with a self antigen can be achieved by using modified cells (e.g. red blood cells (RBC)), or attenuated enveloped viruses. Cells can be modified to express foreign antigens on the surface membrane, allowing co-recognition of the foreign antigen and a self antigen. Without wishing to be bound by theory, it is believed that a mechanism for attenuated enveloped viruses to allow co-recognition of the foreign antigens is by first binding to specific surface receptors of the target host cell membrane and fuse the viral and cell membranes. This is accompanied by delivery of nucleic acids into the host cell, (transcription if the virus is a DNA virus) and translation thereby expressing the foreign, antigen on the surface of the infected host cell.

Accordingly, the invention provides in a first aspect a mammalian cell or a cell membrane containing fraction thereof comprising a viral, bacterial, parasitic or fungal antigen at its cell surface.

Further, the invention also provides a live attenuated enveloped virus comprising a *Plasmodium* antigen or a nucleic acid sequence encoding said *Plasmodium* antigen.

The present invention also provides a live attenuated enveloped virus comprising a tumor-associated antigen or a nucleic acid sequence encoding said antigen.

A cell according to the invention can be used for vaccination against an infectious disease by comprising the foreign antigen at the surface of the cell or by attaching it to the surface of the cell. The cell may comprise a single viral, bacterial, parasitic or fungal antigen or multiple viral, bacterial, parasitic or fungal antigens or combinations thereof. For example the cell may comprise one or more antigen of the same virus, bacterium, parasite of fungus for vaccination against a single pathogen. Alternatively, the cell or fragment thereof may comprise antigens of different pathogens for vaccination against different pathogens. E.g. antigens of different viruses can be combined. As another example, one or more viral antigens can be combined with one or more bacterial antigen for vaccination against multiple pathogens. In one preferred embodiment, the cell or fragment thereof comprises one or more antigens from the same virus, bacterium, parasite of fungus.

The viral, bacterial, parasitic or fungal antigen is preferably expressed at the surface of the cell, preferably the antigen is expressed on the cell membrane. The antigen can also be attached to the cell surface, e.g. by coupling the antigen to a membrane protein or directly inserting it in the cell membrane providing a cell anchor. This can be achieved by any method known in the art to attach proteins or peptides to a cell membrane, including using click chemistry, inteins, or lipophilic protein/peptide comprising a membrane anchor.

It is further preferred that the viral, bacterial, parasitic or fungal antigen is naturally expressed on the surface of the relevant virus, bacterium, parasite or fungus, preferably a membrane protein of a pathogen, which has the advantage that the pathogen can be recognized by host immune cells after infection.

The cell further comprises endogenous antigens to allow co-recognition of the foreign antigen and a self antigen by host receptors on immune cells.

The term "mammalian cell" as used herein refer to any cell type derived from a mammal such as human, non-human primates, or rodent cells. Preferably a cell is used that is endogenous to the subject that is vaccinated. E.g. cells used in human vaccines are preferably human cells, cells used in vaccines for dogs are preferably canine cells, cells used in vaccines for cats are preferably feline cells, etc. In a preferred embodiment, the mammalian cells are human cells. The cells can be autologous but this is not a requirement. Preferred example of cells are stem cells, red blood cells, thrombocytes, white blood cells, neutrophils, eosinophils, basophils, lymphocytes, platelets, nerve cells, neuroglial cells, muscle cells, cartilage cells, bone cells, osteoclasts, osteoblasts, osteocytes, lining cells, skin cells, endothelial cells, epithelial cells, fat cells, reproductive system cells, any mammal derived cell lines or precursors thereof. Preferably the cells are selected from the group consisting of RBCs or a precursor thereof such as proerythroblast (pronormoblast), erythroblast (early normoblast), intermediate normoblast, late normoblast or reticulocyte and thrombocytes or a precursor thereof, such as a megakaryocyte. More preferably, the cells is a RBC or precursor thereof, more preferably a RBC, a erythroblast or a reticulocyte. In a particularly preferred embodiment the mammalian cells are RBC. As described in the examples, RBC and precursors can be advantageously prepared from stem cells, provided with factors that promote differentiation of the stem cells to RBCs. The antigen can be advantageously introduced into the stem cells in amounts that are sufficient for vaccine production. The antigen is for instance introduced together with the factors that promote differentiation to RBCs. Hence, in a further preferred embodiment, the cells, preferably RBC or a precursor thereof, can be produced from induced pluripotent stem cell (iPSC), as these are easy to culture and suitable for large scale production. A combination of different cell types and pathogenic antigens can be used. For example a mix of RBC and/or thrombocytes comprising a viral, bacterial, parasitic and/or fungal antigen at their surface. Thus, multiple cell types and pathogenic antigens can be combined to increase the frequency of recognition by the hosts immune cells and to make vaccinations against multiple targets possible.

The term "cell membrane containing fraction" as used herein refers to any cell fraction comprising one or more membrane proteins, lipids and one or more self antigens. Such fraction can be prepared from cells according to the invention using methods well known in the art, such as by cell disruption or cell fractionation, e.g. by centrifugation of cells with buffer containing e.g. detergents. Cell disruption and fragmentation provide a suspension of membrane fragments/vesicles that contains membrane proteins. Protocols and buffers are commercially available.

The term "antigen" as used herein refers to any molecule with one or more epitopes that stimulates a host's immune system to make a secretory, humoral and/or cellular antigen-specific immune response. An antigen is preferably a protein or peptide.

The term "self antigen" as used herein refers to any molecule with one or more epitopes from an organism that may stimulate an immune response in a different organism but to which the healthy immune system of the parent organism is tolerant. Thus, a self antigen can be any endogenous molecule found on the surface of the host cells or in the cell membrane of the host, or from the same species. For instance, a self antigen is a protein or peptide encoded by the hosts DNA. It preferably excludes antigens of tumor cells.

Examples of antigens that can be used for vaccination against pathogens are viral, bacterial, parasitic or fungal proteins, fractions of a protein, peptides, fusion proteins, glycosylated proteins, compounds comprising amino acids and/or combinations thereof. Examples of infectious viral diseases include but are not limited to smallpox measles, mumps, rubella, chicken pox, hepatitis, herpes, polio, rabies, Ebola, HIV, Severe acute respiratory syndrome (SARS), COVID-19, dengue fever, Zika, and Epstein-Barr. Examples of infectious bacterial diseases include but are not limited to cholera, diphtheria, tetanus, dysentery, bubonic plague, pneumonia, tuberculosis, typhoid, typhus. Example of fungus infections include but are not limited to tinea pedis (athlete's Foot), tinea corporis (ringworm), onychomycosis, tinea versicolor and cutaneous candidiasis. Examples of parasitic infection include but are not limited to malaria, giardiasis, coccidiosis, Leishmaniasis, rhinosporidiosis, trypanomiasis and chagas disease.

The antigen can be any viral, bacterial, parasitic or fungal antigen. Preferably the antigen is an antigen of a virus, bacterium, fungus or parasite that is capable of causing disease in a host, such as a mammal, including a human. Hence, the antigen is preferably a pathogenic antigen, i.e. an antigen of a pathogenic virus, bacterium, fungus or parasite. For instance, antigens of pathogens for which vaccinations are included in national vaccination programs can be expressed on, or attached to, the surface of the mammalian cell. Examples of vial antigens are influenza virus, coronavirus, vaccinia, measles (Rubeola) virus, mumps virus, rubella virus, smallpox virus, ebola virus, or Hepatitis A, B or C virus antigen, preferably surface antigen. Examples of bacterial antigens are Corynebacterium (e.g. diphtheria), Clostridium (e.g. tetanus), Bordetella (e.g. pertussis), Vibrio (e.g. cholera) or Mycobacterium (e.g. tuberculosis) antigen, preferably surface antigen. Examples of parasitic antigens are Trichomonas, Plasmodium, Trypanosoma, Giardia, Entamoeba and Leishmania antigen, preferably surface antigen. Examples of fungal antigens are antigen, preferably surface antigen.

In a preferred embodiment, the cell or fraction thereof according to the invention comprises a *Plasmodium* antigen. In another preferred embodiment, the cell is selected from the group consisting of a red blood cell or a precursor thereof and a thrombocyte or a precursor thereof, more preferably a RBC, and comprises a *Plasmodium* antigen at its cell surface. As shown in the examples, patients naturally infected with malaria show strong afucosylated IgG response against *Plasmodium falciparum* erythrocyte membrane protein-1 (PfEMP1), an antigenic protein expressed on surface of RBCs of patients upon infection with *Plasmodium falciparum.* The *Plasmodium* antigen can be any antigen derived from a pathogenic *Plasmodium* parasite, preferably of a *Plasmodium* parasite that is capable of causing malaria. Various forms of malaria exist, for example, malaria tropica is induced by infection with *Plasmodium falciparum* while malaria tertiana is induced by *Plasmodium vivax. Plasmodium falciparum* and *Plasmodium vivax* are the most common types of *Plasmodium.* Also included are other less common *Plasmodium* parasites such as *Plasmodium knowlesi, Plasmodium malariae* and *Plasmodium ovale.* Hence, the *Plasmodium* antigen is preferably a *Plasmodium falciparum, Plasmodium vivax, Plasmodium knowlesi, Plasmodium malariae* or *Plasmodium ovale* antigen. Preferably the *Plasmodium* antigen is a *Plasmodium falciparum* or *Plasmodium vivax* antigen. Preferably, the *Plasmodium* antigen is an antigen that is expressed on the surface of the *Plasmodium* parasite or the surface of a cell that the parasite can reside in (such as RBC and hepatocytes), preferably a malaria variant surface antigen. More preferably, the *Plasmodium* antigen is selected from the group consisting of *P. falciparum* erythrocyte membrane protein 1 (PfEMP1), pregnancy associated functional and/or structural homologs of PfEMP1 such as VSAPAM (VAR2CSA) or functional homologues thereof, malaria surface targets on various stages of development including *P*. *falciparum* Schizont Egress Antigen-1 (PfSEA-1), *P. falciparum* reticulocyte-binding protein homologue 5 (PfRH5), RIFIN, STEVOR, SURFIN, GLURP, Duffy binding protein (DBP), liver stage antigen 1 (LSA1), Merozoite surface protein 1 (MSP1), *P. falciparum* sporozoite surface protein 2 (PfSSP2), Circum-Sporozoite Protein (CSP), serine repeat antigen and AMA-1, PfMC-2TM, VIR, KIR and SICAvar. More preferably, the *Plasmodium* antigen is selected from the group consisting of malaria variant surface antigens RIFIN, STEVOR, SURFIN, PfEMP1 and pregnancy associated variants such as VSAPAM. More preferably, the *Plasmodium* antigen is selected from the group consisting of P. *falciparum* erythrocyte membrane protein 1 (PfEMP1), RIFIN, STEVOR, SURFIN, GLURP, PfMC-2TM, VIR, KIR, SICAvar and pregnancy associated variants such as VSAPAM or functional homologues thereof. More preferably, the *Plasmodium* antigen is selected from the group consisting of malaria variant surface antigens RIFIN, STEVOR, SURFIN, PfEMP1 and pregnancy associated variants such as VSAPAM. The live attenuated enveloped virus may further comprise multiple different *Plasmodium* antigens. Other antigens that may be used are VAR6 which is expressed in general malaria cases and VAR2CSA which is expressed solely by parasites in pregnancy associated malaria. Further, non-surface malaria antigens such as the glutamine-rich protein (GLURP) antigen expressed almost exclusively on the extracellular stage of the parasite (merozoites) may also be co-expressed with e.g. a signal peptide to drive expression of the antigen to the cell surface.

In a particularly preferred embodiment is provided a RBC or cell membrane containing fraction thereof expressing a *Plasmodium* antigen at its cell surface.

A cell according to the invention can be generated by any method known in the art to express an exogenous antigen at its surface, e.g. by introducing a nucleic acid encoding said antigen in the cell or by chemical modifications. Introduction of exogenous nucleic acid constructs into mammalian cell to express the encoded peptide or protein has become routine experimentation during the last decades, including expression thereof at the cell surface. Hence, a person skilled in the art is well capable of generating a cell according to the invention using his common general knowledge. Examples of well known methods for introducing a nucleic acid in a cell include transfection using e.g. cationic compounds with a construct or plasmid DNA comprising the nucleic acid sequence encoding the antigen, by transduction using recombinant viral vectors such as a lentiviral vector or a retroviral vector, or a transposon comprising the nucleic acid sequence encoding the antigen. The construct used to introduce the nucleic acid into the cell may comprise one or more additional regulatory sequences, such as signal sequences of membrane proteins that drive expression of the antigen on the cell membrane, and/or an inducible promoter or constitutive promoter, preferably specific for the mammal from which the cell is derived, to drive expression of the antigen.

In a preferred embodiment, the mammalian cell is a RBC or a precursor thereof, preferably a RBC, erythroblast or reticulocyte. RBCs are for instance obtained from blood of one or more donors. Alternatively, RBCs are prepared from other cell types, e.g. from mammalian, preferably human, induced pluripotent stem cells (iPSC) or from mammalian, preferably human, peripheral blood mononuclear cells (PBMCs). Several methods can be used for generation of RBC or precursors thereof, that can be provided with an antigen in accordance with the present invention. For example, known in the art is generation of enucleated RBC from cord blood CD34+ cells. As described in example 2 and in Theil et al. (Hum. Mol Gen. 2017; 26(23): 4989-4698), iPSCs derived from fibroblasts or peripheral blood mononuclear cells (PBMC) can also be induced into embryoid bodies colonies and differentiated into CD71+/CD235+erythroblasts and mature RBC. The antigens can be introduced for instance in the erythroblast stage or at the PBMC stage. Further, RBC can also be generated from adult PBMC as described by Heshusius et al. Blood Adv. 2019 Nov 12; 3(21): 3337-3350. A cell according to the invention may express the surface antigen transiently but also stable cell lines expressing the surface antigen can be prepared. The nucleic acid molecule or vector may be introduced into the cell by any method known in the art, such as by lentiviral transduction, retroviral transduction, DNA electroporation, or RNA electroporation. Methods for transduction or electroporation of cells with a nucleic acid are known to the skilled person.

As demonstrated in the examples, live attenuated virus can also be used to induce an afucosylated immune response in a subject. Accordingly, the invention provides a live attenuated enveloped virus comprising a *Plasmodium* antigen or a nucleic acid sequence encoding said *Plasmodium* antigen. The *Plasmodium* antigen can be any antigen derived from a pathogenic *Plasmodium* parasite, preferably of a *Plasmodium* parasite that is capable of causing malaria. Various forms of malaria exist, for example, malaria tropica is induced by infection with *Plasmodium falciparum* while malaria tertiana is induced by *Plasmodium vivax. Plasmodium falciparum* and *Plasmodium vivax* are the most common types of *Plasmodium.* Also included are other less common *Plasmodium* parasites such as *Plasmodium knowlesi, Plasmodium malariae* and *Plasmodium ovale.* Hence, the *Plasmodium* antigen is preferably a *Plasmodium falciparum Plasmodium vivax, Plasmodium knowlesi, Plasmodium malariae* or *Plasmodium ovale* antigen. Preferably the *Plasmodium* antigen is a *Plasmodium falciparum* or *Plasmodium vivax* antigen. Preferably, the *Plasmodium* antigen is an antigen that is expressed on the surface of the *Plasmodium* parasite or on the surface of a cell that the parasite can reside in (such as RBC and hepatocytes), preferably a malaria variant surface antigen. More preferably, the *Plasmodium* antigen is selected from the group consisting of *P. falciparum* erythrocyte membrane protein 1 (PfEMP1), pregnancy associated functional and/or structural homologs of PfEMP1 such as VSAPAM (VAR2CSA) or functional homologues thereof, malaria surface targets on various stages of development including *P. falciparum* Schizont Egress Antigen-1 (PfSEA-1), *P*. *falciparum* reticulocyte-binding protein homologue 5 (PfRH5), RIFIN, STEVOR, SURFIN, GLURP, Duffy binding protein (DBP), liver stage antigen 1 (LSA1), Merozoite surface protein 1 (MSP1), *P. falciparum* sporozoite surface protein 2 (PfSSP2), Circum-Sporozoite Protein (CSP), serine repeat antigen and AMA-1, PfMC-2TM, VIR, KIR and SICAvar. More preferably, the *Plasmodium* antigen is selected from the group consisting of malaria variant surface antigens RIFIN, STEVOR, SURFIN, PfEMP1 and pregnancy associated variants such as VSAPAM. More preferably, the *Plasmodium* antigen is selected from the group consisting of P. *falciparum* erythrocyte membrane protein 1 (PfEMP1), RIFIN, STEVOR, SURFIN, GLURP, PfMC-2TM, VIR, KIR, SICAvar and pregnancy associated variants such as VSAPAM or functional homologues thereof. More preferably, the *Plasmodium* antigen is selected from the group consisting of malaria variant surface antigens RIFIN, STEVOR, SURFIN, PfEMP1 and pregnancy associated variants such as VSAPAM. The live attenuated enveloped virus may further comprise multiple different *Plasmodium* antigens. Other antigens that may be used are VAR6 which is expressed in general malaria cases and VAR2CSA which is expressed solely by parasites in pregnancy associated malaria. Further, non-surface malaria antigens such as the glutamine-rich protein (GLURP) antigen expressed almost exclusively on the extracellular stage of the parasite(merozoites) may also be co-expressed with e.g. a signal peptide to drive expression of the antigen to the cell surface.

A live attenuated enveloped virus can also be used for vaccination against cancer cells where induction of an afucosylated IgG response resulting in increased ADCC can also be advantageous.

Accordingly, the invention also provides a live attenuated enveloped virus comprising a tumor-associated antigen or a nucleic acid sequence encoding said antigen. One major challenge in cancer vaccines is the inherently-weak immunogenicity of tumor-associated antigens compared to for example viral proteins that are highly-immunogenic. Tumor-associated antigens are produced by tumor cells and can trigger the host's immune system against cancer cells, which make them useful for treatment of cancer. However, when provoking an immune response using viral vectors comprising tumor-associated antigens, the weaker epitopes derived from tumor-associated antigens may be ignored by the adaptive immune response based on a biased focus on xenogeneic viral proteins. Afucosylated IgG variants as anti-cancer therapeutic antibodies have shown enhanced effector function against cancer cells through the Fc-mediated immune effector function ADCC. However directly inducing an afucosylated IgG response in a subject against cancer cells is unknown in the art. This can now be achieved with a live attenuated enveloped virus comprising a tumor-associated antigen according to the invention.

The term "tumor-associated antigen" as used herein refers to any molecule (such as a protein, peptide, polypeptide, lipoprotein, lipopeptide, glycoprotein, lipid, glycolipid, etc.) that is expressed inside or on the surface of tumor cells and that are able to evoke an immune responses against the tumor cells when expressed on the surface of an antigen presenting cell. Tumor associated antigens are preferably preferentially presented by tumor cells over non-tumor cells and thus allow to distinguish tumor cells and non-tumor cells. They often differ from non-tumor counterparts by modification in the molecule, such as mutations, insertions and deletions, or by different levels of expression, generally or in other stages of development. The tumor associated antigen is preferably expressed on the cellular surface of the tumor cell. Preferably, the tumor associated antigen comprises a protein or peptide, more preferably the tumor associated antigen is a protein or peptide.

The tumor can be any type of tumor known in the art such as carcinoma, sarcoma, melanoma, lymphoma, and leukemia. Example of tumor-associated antigens include peptides, glycopeptides, phosphopeptides, viral peptides, and peptides resulting from common mutations in oncogenes and tumor-suppressor genes, or common gene fusion events. Also tumor-associated antigens that have been tested as candidates for anticancer vaccines are included such as cancer testis antigens, tissue specific antigens, oncofetal antigens, differentiation antigens, growth factors, growth factor receptors, adhesion factors, signal transduction proteins, transcription factors, oncogene products, tumor suppressor gene products, microbial agents, and the like. Examples include, but are not limited to, antigens selected from the group consisting of an SSX protein, SSX-2, SSX-4, a MAGE protein, MAGE-1, MAGE-3, PRAME, NY-ESO-1, LAGE, PSMA, PSCA, ' SCP-1, melan-AlMART-1, AFP, CEA, CA-125, MUC-1, ETA, 707-AP, ART-4, a CAMEL, CAP-1, CASP-8, CDC27m, and CDK4/m.

The term "live attenuated virus" as used herein refers to a live, altered form or strain of a virus which is less virulent than the parent form or strain. Live attenuated viruses can be generated by any method known in the art. For example, by repeatedly passaging a virus in cultured cells followed by selection or by recombinant DNA technologies including deleterious gene mutation, altered replication fidelity, codon deoptimization, control by microRNAs or gene editing technologies such as zinc finger nucleases or CRISPR-Cas. The live attenuated virus may still able to replicate but this is not a requirement.

The term "enveloped virus" as used herein refers to any pathogenic or non pathogenic virus that is enveloped with a lipid bilayer, and infect their target cells by inducing the fusion of the viral envelope with the cell membrane. The virus can be any enveloped viruses known in the art but preferably the virus is selected from the group consisting of herpes simplex virus, varicella-zoster virus, poxyvirus (e.g. Modified Vaccinia Ankara), cytomegalovirus, Epstein-Barr virus, vaccinia virus, Dengue virus, hepatitis virus, yellow fever virus, influenza virus, mumps virus, respiratory syncytial virus, retrovirus and coronavirus. More preferably, the virus virus is selected from the consisting of cytomegalovirus, measles virus, mumps virus or Hepatitis B virus.

The terms "pathogenic" and "virulent" are used interchangeably herein to describe an organism or a strain thereof which causes disease (i.e. a pathogen) in a susceptible host.

The term "nucleic acid encoding an antigen" refers to any nucleic acid molecule such as ribonucleic acid (RNA) or deoxyribonucleic acid (DNA) that encodes a viral, bacterial, parasitic or fungal antigen. The nucleic acid molecules can be either synthesized or directly derived from cells or tissue lysates and can further be incorporated into a variety of expression systems known to those of skilled in the art. The nucleic acid is preferably introduced into a live attenuated virus.

A live attenuated virus according to the invention can be generated by any method known in the art to comprise an exogenous antigen and express at its surface. Examples of well known methods for introducing a nucleic acid in a virus include transduction using with a construct or plasmid DNA comprising the nucleic acid sequence encoding the antigen or by using recombinant viral vectors such as a lentiviral vector or a retroviral vector, a transposon comprising the nucleic acid sequence encoding the antigen, or gene editing technologies such as CRISPR/Cas. The sequence preferably comprises a promoter, a signal sequences that drive expression of the antigen on the cell membrane, and a poly-A signal sequence. The nucleic acid molecule or vector may be introduced into the virus by any method known in the art, such as by lentiviral transduction, retroviral transduction, DNA electroporation, or RNA electroporation.

The cell or fraction thereof or a live attenuated enveloped virus according to the invention is capable of stimulating an afucosylated antigen-specific IgG response in a subject. The antigen-specific IgGs are specific for the antigen or antigens that are comprised by the cell or fraction thereof or the live attenuated virus according to the invention. The term "specific for" as used herein refers to the interaction between an antibody and its antigen. The term means that said antibody preferentially binds to said antigen over other antigens. Although the antibody may non-specifically bind to other amino acid sequences or antigens, the binding affinity of said antibody or part for its epitope is significantly higher than the non-specific binding affinity of said antibody to other amino acid sequences or antigens and generally non-measurable.

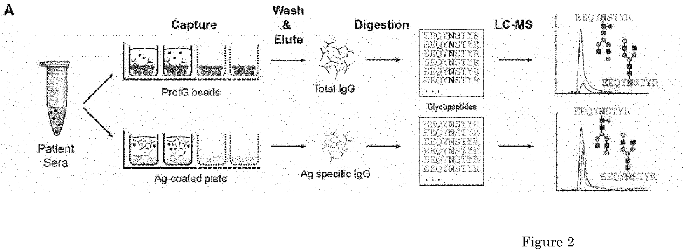

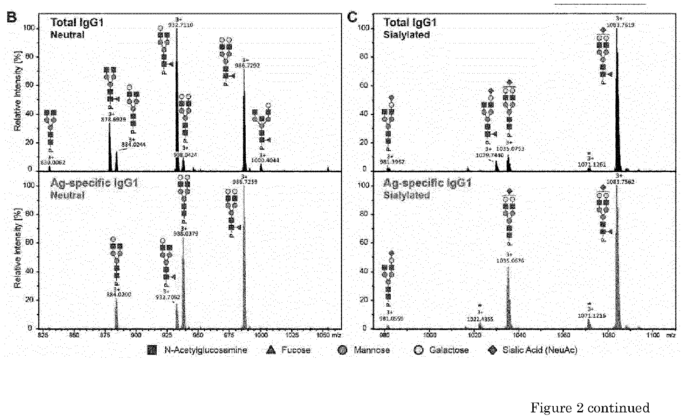

The term "stimulating an afucosylated IgG response" as used herein refers to the ability to induce production of antigen-specific IgG antibodies in a subject lacking the core fucose moiety in the Fc glycan. As a result the population of antibodies specific for the antigen contains a higher proportion of antibodies that lack this fucose in one or both of the Asn 297 glycans as compared to the total IgG population in the subject. Figure 2 schematically shows the composition of the Asn297 glycan moieties. Each antibody comprises two of the Asn297 glycans. Hence, each antibody can have either two afucosylated glycans, one afucosylated glycan and one fucosylated glycan, or two fucosylated glycan. Preferably the proportion of IgG antibodies wherein at least one of the two glycans lacking a fucose is increased. More preferably, the proportion of IgG antibodies wherein both glycans lack fucose is increased. The term "afucosylated IgG antibodies" as used herein refers to IgG antibodies that lack a fucose in both Asn 297 glycans. The average percentage of afucosylated IgG antibodies (antibodies lacking fucose in both Asn297 glycans) in human is very low from birth (∼0-1%) but this percentage increase with age to an average 6% at the age of 20. The percentage of afucosylated antigen-specific IgG antibodies stimulated with a cell or fraction thereof or a live attenuated enveloped virus according to the invention is preferably more than 6% of the total amount of IgG antibodies specific for said antigen, more preferably more than 7%, more preferably more than 8%, more preferably more than 9%,more preferably more than 10%, more preferably more than 15%, more preferably more than 20%, more preferably more than 30%, more preferably more than 40%, such as more than 50%, more than 60% or more than 70% of the IgG antibodies specific for said antigen. Percentage of afucosylated antigen-specific IgG antibodies of about 90% of IgG antibodies specific for the antigen have been observed.

The term "subject" as used herein refers to an animal, more preferably a mammal, most preferably a human.

Further provided is a pharmaceutical composition comprising a cell or fraction thereof or a live attenuated enveloped virus according to the invention and a pharmaceutically acceptable carrier, diluent and/or excipient.

Also provided is a vaccine comprising a cell or fraction thereof or a live attenuated enveloped virus according to the invention, and a pharmaceutically acceptable carrier, diluent and/or excipient.

In general, any pharmaceutic composition or vaccine which does not interfere with the function of a cell or fraction thereof or a live attenuated enveloped virus according to invention can be used. The pharmaceutical composition or vaccine is preferably suitable for human use. Examples of suitable carriers comprise a solution, lactose, starch, cellulose derivatives and the like, or mixtures thereof. In a preferred embodiment said suitable carrier is a solution, for example saline. For making dosage units, e.g. tablets, the use of conventional additives such as fillers, colorants, polymeric binders and the like, is contemplated. Examples of excipients which can be incorporated in tablets, capsules and the like are the following: a binder such as gum tragacanth, acacia, corn starch or gelatin; an excipient such as microcrystalline cellulose; a disintegrating agent such as corn starch, pregelatinized starch, alginic acid and the like; a lubricant such as magnesium stearate; a sweetening agent such as sucrose, lactose or saccharin; a flavoring agent such as peppermint, oil of wintergreen or cherry. Compositions for intravenous administration may for example be solutions comprising an attenuated enveloped virus of the invention in sterile isotonic aqueous buffer. The intravenous compositions may include for instance solubilizing agents, stabilizing agents and/or a local anesthetic to ease the pain at the site of the injection.

The cell or fraction thereof or a live attenuated enveloped virus according to the invention for systemic administration, preferably for parenteral administration, including but not limited to intravenous, intramuscular and subcutaneous administration, or for oral administration, including but not limited to tablets, capsules, liquids, emulsions, suspensions.

The pharmaceutical composition or vaccine may further comprise several components such as stabilizers, adjuvants, antibiotics, and preservatives and can be administered to a subject at any therapeutically relevant amount.

The vaccines can further comprise one or more adjuvants that are pharmaceutically acceptable. Many such adjuvants are known in the art. Adjuvants are substances that enhance the immune response to antigens, but are not necessarily immunogenic themselves. A wide range of adjuvants provokes potent immune responses to antigens, including aluminum hydroxide, aluminum phosphate, saponins complexed to membrane protein antigens (immune stimulating complexes), pluronic polymers with mineral oil, killed Mycobacteria in mineral oil, Freund's complete adjuvant, bacterial products, such as muramyl dipeptide (MDP) and lipopolysaccharide (LPS), as well as lipid A.

Further provided is a cell or fraction thereof or a live attenuated enveloped virus according to the invention for use as a vaccine. Further provided is a method of vaccination comprising administering to the subject a therapeutically effective amount of a cell or fraction thereof or a live attenuated enveloped virus according to the invention.

Also provided is a cell or fraction thereof or a live attenuated enveloped virus according to the invention for use in the treatment or prevention of a viral, bacterial, parasitic or fungal infection. Also provided is a method for treatment or prevention of a viral, bacterial, parasitic or fungal infection comprising administering to a subject a therapeutically effective amount of a cell or fraction thereof or a live attenuated enveloped virus according to the invention. Also provided is a use of a cell or fraction thereof or a live attenuated enveloped virus according to the invention for the manufacture of a medicament for the treatment or prevention of a viral, bacterial, parasitic or fungal infection. The cell or fraction thereof or a live attenuated enveloped virus which comprises an heterologous antigen can be used for treatment or prevention of a disease associated with the pathogen from which said antigen is derived. Thus, if the antigen is a *Plasmodium* antigen, it can be used for treatment or prevention of malaria. If the antigen is a *Hepatitis B* antigen, it can be used for treatment or prevention of Hepatitis B infection. If the antigen is a *Mycobacterium tuberculosis* antigen, it can be used for treatment or prevention of tuberculosis etc.

Further provided is a cell or fraction thereof or a live attenuated enveloped virus according to the invention comprising a *Plasmodium* antigen for use in the treatment or prevention of malaria. Further provided is a method for treatment or prevention of malaria comprising administering to a subject a therapeutically effective amount of a cell or fraction thereof or a live attenuated enveloped virus according to the invention comprising a *Plasmodium* antigen. Further provided is a use of a cell or fraction thereof or a live attenuated enveloped virus according to the invention comprising a *Plasmodium* antigen for the manufacture of a medicament for the treatment or prevention of malaria.

Also provided is a live attenuated enveloped virus comprising a tumor-associated antigen according to the invention for use in the treatment or prevention of cancer. Further provided is a method for treatment of cancer comprising administering to a subject a therapeutically effective amount of a live attenuated enveloped virus according to the invention. Further provided is use of a live attenuated enveloped virus according to the invention for the manufacture of a medicament for the treatment of cancer. Immunization with said live attenuated enveloped virus lead to expression of the tumor-associated antigen on the surface of the infected host cell, allowing co-recognition by the host's immune cells in combination with a self antigen, thereby inducing an afucosylated IgG response.

The cancer can be any type of cancer including primary tumors, secondary tumors, advanced tumors and metastases. Non-limiting examples tumors that can be treated or prevented in accordance with the invention are acute myeloid leukemia (AML), chronic myeloid leukemia (CML), chronic lymphocytic leukemia (CLL), acute lymphoblastic leukemia (ALL), chronic myelomonocytic leukemia (CMML), lymphoma, multiple myeloma, eosinophilic leukemia, hairy cell leukemia, Hodgkin lymphoma, non-Hodgkin lymphoma, large cell immunoblastic lymphoma, plasmacytoma, lung tumors, small cell lung carcinoma, non-small cell lung carcinoma, pancreatic tumors, breast tumors, liver tumors, brain tumors, skin tumors, bone tumors, colon tumors, rectal tumors, anal tumors, tumors of the small intestine, stomach tumors, gliomas, endocrine system tumors, thyroid tumors, esophageal tumors, gastric tumors, uterine tumors, urinary tract tumors and urinary bladder tumors, kidney tumors, renal cell carcinoma, prostate tumors, gall bladder tumors, tumors of the head or neck, ovarian tumors, cervical tumors, glioblastoma, melanoma, chondrosarcoma, fibrosarcoma, endometrial, esophageal, eye or gastrointestinal stromal tumors, liposarcoma, nasopharyngeal, thyroid, vaginal and vulvar tumors. A skilled person is well capable of selecting an appropriate tumor associated antigen that can be used for treatment of the relevant tumor or cancer.

As used herein, the term "prevention" refers to preventing or delaying the onset of a disease and/or the appearance of clinical symptoms of the disease in a subject that does not yet experience clinical symptoms of the disease. The term "treatment" refers to inhibiting the disease, i.e., halting or reducing its development or at least one clinical symptom thereof, and to relieving symptoms of the disease.

Further provided is a cell or fraction thereof or a live attenuated enveloped virus according to the invention for use in inducing and/or stimulating an afucosylated IgG response against an antigen of interest in a subject, wherein said cell or fraction thereof comprises said antigen of interest at its cell surface, or said virus comprises said antigen of interest or a nucleic acid encoding said antigen of interest, preferably wherein said afucosylated IgG molecules have increased binding to the FcyRIIIa and/or FcyRIIIb receptor.

Further provided is a cell or fraction thereof or a live attenuated enveloped virus according the invention for use in inducing an immune response against an antigen of interest in a subject, wherein said cell or fraction thereof comprises said antigen of interest at its cell surface, or said virus comprises said antigen of interest or a nucleic acid encoding said antigen of interest.

The term "immune response" refers to the development of a humoral immune response, a cellular immune response, or a humoral and a cellular immune response to an antigen. A "humoral immune response" refers to an immune response that is mediated by antibodies. A "cellular immune response" is one mediated by T-lymphocytes or other white blood cells such as B cells or both, and includes the production of cytokines, chemokines and similar molecules produced by activated T-cells, white blood cells, or both. Immune responses can be determined using standard immunoassays and neutralization assays, which are known in the art.

The term "inducing/stimulating" as used herein refers to the action of generating, initiating, promoting, forming, regulating, activating, enhancing or accelerating a biological process. An example is a *Plasmodium* antigen recognized by the hosts immune cells that's subsequently trigger a cascade of biological process (immune response) in the host.

An "antigen of interest" refers to any viral, bacteria, parasitic, fungal, or tumor-associated antigens that can be expressed in a cell or fraction thereof or an attenuated virus according to the invention. The antigen of interest is of a virus, bacterium, parasite, fungus or tumor against which vaccination is desired.

The term "increased binding" " refers to the determination any techniques known in the art that an antigen recognizes and binds with an increased affinity to a given receptor. Such standard techniques includes ELISA, equilibrium dialysis, gel filtration, and the monitoring of spectroscopic changes that result from binding, e.g. using fluorescence anisotropy, either by direct binding measurements or competition assays with another binder. Examples of increased binding is an afucosylated IgG antibody having increased IgG-Fc-receptor IIIa (FcγRIIIa) and FcyRIIIb affinity

Features may be described herein as part of the same or separate aspects or embodiments of the present invention for the purpose of clarity and a concise description. It will be appreciated by the skilled person that the scope of the invention may include embodiments having combinations of all or some of the features described herein as part of the same or separate embodiments.

The invention will be explained in more detail in the following, non-limiting examples.

### Brief description of the drawings

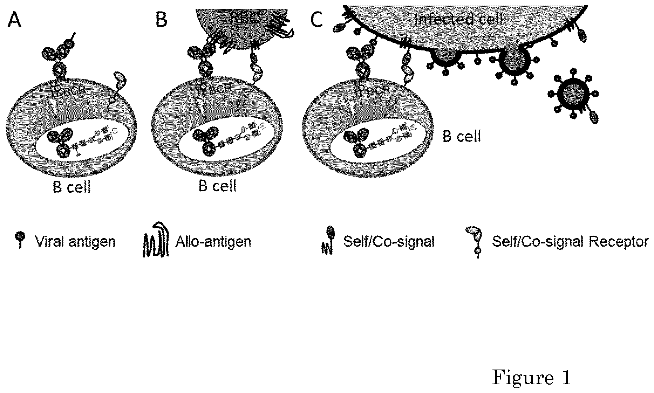

**Figure 1****: Hypothetical model explaining how the context of antigen can lead to altered immune signaling, giving rise to altered IgG-glycosylation** A) Immune response to soluble protein antigen: B-cell receptor (BCR, a membrane immunoglobulin) is activated, resulting in the production of normal fucosylated antibodies. B) Immune response to allo-antigens: Paternal allo-antigen on a Red Blood Cell (RBC) recognized by the BCR, and possibly by a yet unknown immune regulatory receptor-ligand pair providing a signal for self. C) Immune response to enveloped viral infection: Recognition of enveloped-virus infected cells by B cells is similar as for cellular-alloantigen recognition. The initial recognition can potentially occur towards enveloped-virally infected cells and possibly after viral assembly (far right). The proposed signaling in b-c) causes altered glyco-programming of the B cells culminating in a unique IgG-response characterized by a low fucosylation (fucose red triangle) and enhanced ADCC. The model potentially explains both why immune response to soluble proteins, non-enveloped viruses and cellular pathogens such as bacteria is different to responses to enveloped-viruses and why immune responses to allo-antigens mimic that of an enveloped viral infection.
**Figure 2****: Flowchart of antibody specific IgG1 glycosylation analysis and mass spectrometric analysis. A)** Antibodies from sera were captured using ProteinG beads and antigen-coated 96-well plates resulting in total and antigen-specific IgG fractions, respectively. Thereafter, isolated IgGs were digested with trypsin and the resulting glycopeptides were analyzed by nano liquid chromatography-coupled mass spectrometry. **B,C)** Representative mass spectra of glycopeptides encompassing the Fc glycosylation site Asn297. Neutral **(B)** and sialylated **(C)** IgG1 glycopeptides are shown from a single patients' total (upper panel, in black) and antigen-specific (lower panel, in red) IgG1 fraction. Asterisks indicate non-Fc glycopeptides.

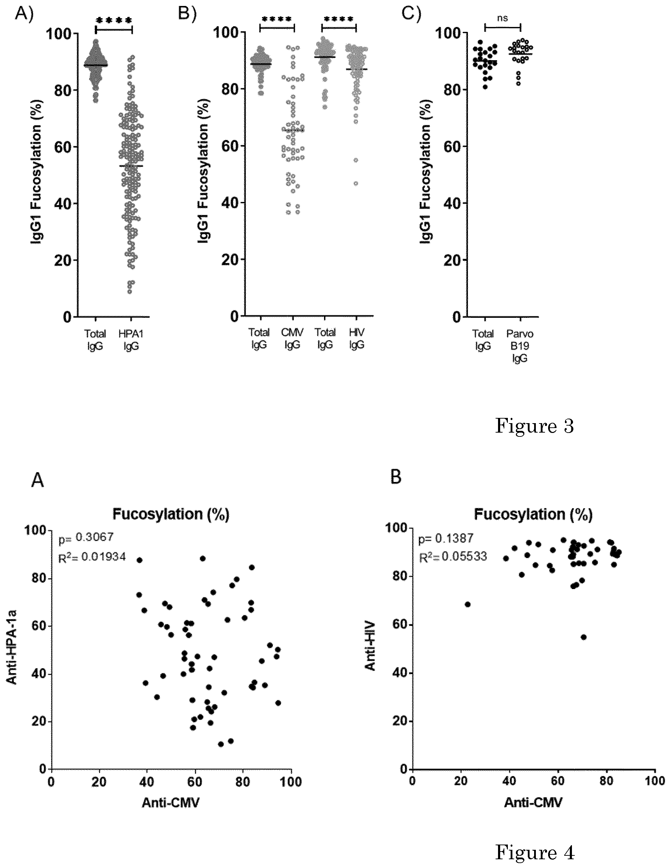

**Figure 3****: Foreign membrane protein antigens, such as envelope proteins of enveloped viruses or alloantigens can trigger afucosylated immune responses.** IgG1-Fc Fucosylation levels of total (filled circles) and antigen-specific (open circles) antibodies are shown for each differently color-coded group of antigens: **A)** alloantigen HPA-1a; **B)** viral envelope antigens from CMV and HIV; **C)** non-enveloped viral antigens from Parvo B19. Statistical analysis was performed with paired t tests (**** = p<0.0001).
**Figure 4****: Antibody fucosylation is not determined by a general host factor. a**. No correlation was found between the level of IgG1 Fc-fucosylation made during alloimmunization against HPAla in pregnancy (y-axis) and CMV (x-axis) in the same individual. **b.** Also no correlation was found between the level of IgG1 Fc-fucosylation made against HIV (y-axis) and CMV (x-axis) in the same individual. Statistical analysis was performed using Pearson correlation.

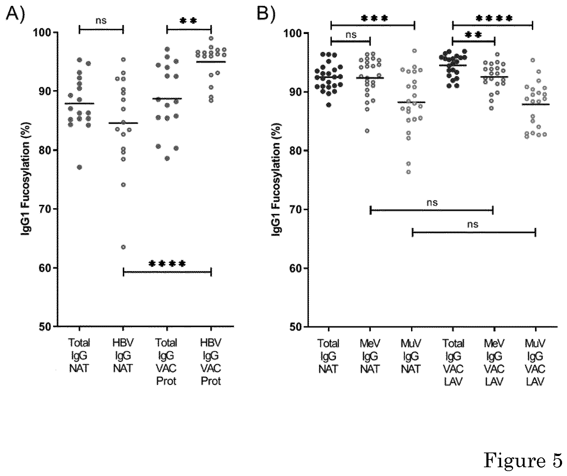

**Figure 5****: IgG afucosylation can be induced by live attenuated viruses. A)** IgG Fc-fucosylation levels of total and antigen-specific IgGs in individuals naturally infected (NAT) with HBV or vaccinated with recombinant soluble HBsAg (VAC Prot). **B)** Fc-fucosylation levels of total and ag-specific IgGs in individuals naturally infected (NAT) with MeV or MuV, or vaccinated with live attenuated viruses (VAC LAV) for MeV and MuV. Statistical analysis was performed with a paired t test for the total vs Ag-specific comparisons and with unpaired t-tests for the rest.**=p<0.01, ***=p<0.001 **** = p<0.0001.

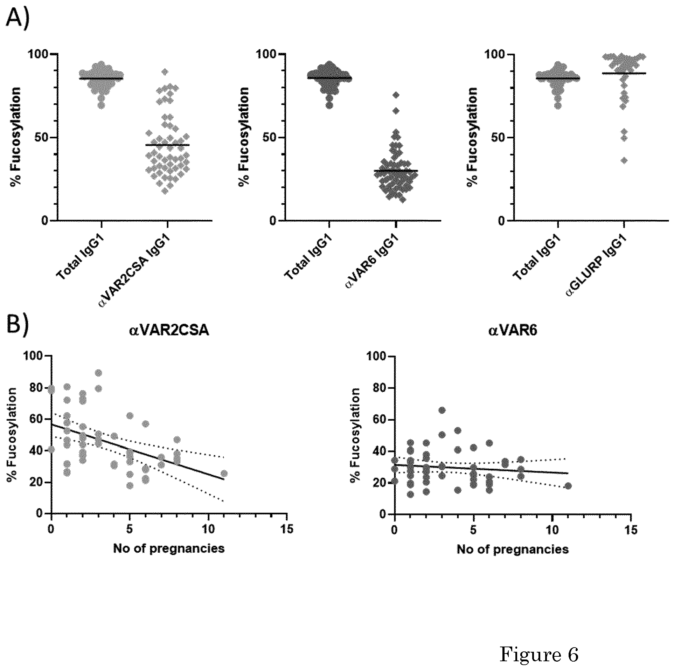

**Figure 6****: Anti RBC-stage malaria responses show strong afucosylated-IgG signatures. A)** Responses to pregnancy-associated VAR2CSA, non-pregnancy associated VAR6 (both expressed on infected RBC) and GLURP (merozoite antigen). **B)** The association between number of pregnancies and fucosylation for the VAR2CSA- and VAR6-specific IgG.

### Examples

### Example 1

### Materials and methods

### Patient samples

Healthy blood donor samples from Sanquin, Amsterdam, The Netherlands, were used to analyze ParvoB19 (n=22), MeV (n=21 natural infection, n=17 LAV), MuV (n=21 natural infection, n=17 LAV) and HBV antibodies (n=17 natural infection, n=16 HBsAg vaccination). Anti-HPA-la samples have been described elsewhere (13). HIV-samples (n=80) from the Amsterdam Cohort Studies on HIV infection and AIDS (ACS) were used to analyze HIV-specific antibody glycosylation. The ACS have been conducted in accordance with the ethical principles set out in the declaration of Helsinki and all participants provided written informed consent. The study was approved by the Academic Medical Center institutional Medical Ethics Committee of the University of Amsterdam.

Peripheral blood samples from patients with HPA-1a alloantibodies and CMV-specific antibodies (n=62) were collected by the Finnish Red Cross Blood service, Platelet Immunology Laboratory, Helsinki, Finland.

Blood from Ghana women were collected, with ethical approval by the Institutional Review Board of the Noguchi Memorial Institute for Medical Research, University of Ghana as described previously (P. Ampomah, L. Stevenson, M. F. Ofori, L. Barfod, L. Hviid, B-cell responses to pregnancy-restricted and -unrestricted Plasmodium falciparum erythrocyte membrane protein 1 antigens in Ghanaian women naturally exposed to malaria parasites. Infect. Immun. 82, 1860-1871 (2014).

### Purification of CMV-specific antibodies from sera

CMV-specific antibodies were purified using antigen-coated plates (Serion ELISA classic, Cytomegalovirus IgG, Wurzburg, Germany). Sera (20 *µ*L) diluted in specimen diluent (80 *µ*L) from kit was incubated for 1 hour at 37 °C degrees. Positive and negative controls from the kit and CMV-negative patients samples were used as controls. The plates were washed seven times: three times with 300 *µ*L wash-buffer from the kit, followed by two washed with the same volume Phosphate buffered saline (PBS) and deionized water. The bound antibodies were then eluted using 100 *µ*L of 100 mmol/l formic acid. No IgG was found in eluates from blank wells and CMV-negative patients samples.

### Purification of MeV- and MuV-specific antibodies from sera

Ag-specific antibodies were purified using antigen-coated plates (Serion ELISA classic, Measles IgG and Mumps IgG, Wurzburg, Germany). Sera (20 *µ*L) diluted in specimen diluent (80 *µ*L) from kit was incubated for 1 hour at 37 °C degrees. Positive and negative controls from the kit were used as controls. The plates were washed seven times: three times with 300 *µ*L wash-buffer from the kit, followed by two washed with the same volume Phosphate buffered saline (PBS) and 50mM ammonium bicarbonate. The bound antibodies were then eluted using 100 *µ*L of 100 mM formic acid. IgG was found in the eluates of positive controls and no IgG was found in eluates from blank wells and negative control samples.

### Purification of HBV-specific antibodies from sera

To isolate HBsAg specific antibodies from patients after infection and vaccination, HBs antigen-coated plates (ETI-AB-AUK-3, Diasorin, Schiphol-Rijk, The Netherlands) were used. Sera were diluted five times in specimen diluent from kit (20 *µ*L serum with 80 *µ*L diluent) and incubated for 1 hour at room temperature with shaking 450 r.p.m. (Heidolph Titramax 100, Schwabach, Germany). HBV-naive and HBV-resolved samples from Sanquin, Amsterdam, The Netherlands were used as controls. Washing and eluting specific antibodies was done as described above for CMV-specific antibodies.

### Purification of HIV-specific antibodies from sera

HIV-specific antibodies were isolated using HIV antigen-coated plates (Murex HIV1.2.0 kit 9E25-01, Diasorin, Schiphol-Rijk, The Netherlands). Sera were diluted two times in sample diluent from kit (50 *µ*L serum with 50 *µ*L diluent) and incubated for 1 hour at room temperature shaking 450 r.p.m. (Heidolph Titramax 100, Schwabach, Germany). As positive control, anti-HIV gp120 monoclonal was used (IgG1b12; 100 *µ*g purified antibody in PBS at 1 mg/ml; NIH Aids Reagent Program, La Jolla, CA, US). Washing and eluting specific antibodies was done as described above for CMV-specific antibodies.

### Purification of malaria antibodies from plasma

VAR2CSA-, VAR6-, and GLURP-specific antibodies were isolated using 96 well Maxisorp plates (NUNC) coated over-night (4 °C) in phosphate-buffered saline (PBS) with recombinant VAR2CSA (2µg/mL), VAR6 (2µg/mL)(23), and GLURP (1µg/mL) protein. Following coating plates were washed 3x with PBS supplemented with TWEEN 20® (0.05%) (PBS-T). Plates were subsequently incubated (1h, rt) with diluted plasma samples (1:10), a monoclonal VAR2CSA-reactive antibody (PAM1.4 (1 µg/mL) and a polyclonal GLURP-reactive IgG preparation (1 µg/mL) all in PBS-T. The samples were initially incubated on the VAR2CSA and VAR6 coated plated, after which the samples were pooled and incubated on the GLURP coated plate. Following the incubation the plates were washed with 3xPBS-T, 2x PBS, and 2x ammonium bicarbonate (50 mM). Antigen-specific antibodies were finally eluted by formic acid (100 mM; 5 min shaking). (

### Purification of ParvoB19-specific antibodies from sera

ParvoB19-specific antibodies were isolated using ParvoB19 antigen-coated plates (Abcam1788650- Anti-Parvovirus B19 IgG ELISA, Cambridge, United Kingdom). Sera were diluted five times in sample diluent from kit (20 *µ*L serum with 80 *µ*L diluent) and incubated for 1 hour at room temperature shaking 450 r.p.m. (Heidolph Titramax 100). Positive and negative controls from the kit were used as controls. Washing and eluting specific antibodies was done as described above for CMV-specific antibodies.

### Purification of total IgG from sera

Total IgG1 antibodies were captured from 2 *µ*L of serum using Protein G Sepharose 4 Fast Flow beads (GE Healthcare, Uppsala, Sweden) in a 96-well filter plate (Millipore Multiscreen, Amsterdam, The Netherlands) as described previously (11) or by using Protein G cartridges on the AssayMAP Bravo (Agilent Technologies, Santa Clara, USA) Briefly, 1 *µ*L serum diluted in PBS were applied to the cartridges, followed by washes of PBS, LC-MS pure water and finally eluted with formic acid (1%).

### Mass spectrometric IgG-Fc glycosylation analysis

Eluates containing either antigen-specific antibodies or total IgG were collected in V-bottom plates, dried by vacuum centrifugation for 2.5 hours at 50°C. The HPA1a, CMV, HIV, ParvoB19 and HBV samples were dissolved in 20 *µ*L of 50 mmol/l ammonium bicarbonate followed by shaking for 10 minutes. Antibody samples were then subjected to proteolytic cleavage using trypsin as described before (11). The MeV and MuV cohort samples were dissolved in a buffer containing 0.4% sodium deoxycholate(SDC), 10mM TCEP, 40mM chloroacetamide, 100 mM TRIS pH8.5. After 10min incubation at 95C, 250 ng Trypsin in 50 mM ammonium bicarbonate was added. The digestion was stopped after an overnight incubation by acidifying to 2% formic acid. Prior to MS injection, SDC precipitates were removed by centrifuging samples at 20 000 rcf for 30 minutes.

Analysis of IgG Fc-glycosylation was performed with nanoLC reverse phase (RP)-electrospray (ESI)- MS on an Ultimate 3000 RSLCnano system (Dionex/Thermo Scientific, Breda, The Netherlands) coupled to an amaZon speed ion trap MS (Bruker Daltonics, Bremen, Germany) (R. Kapur, et al., A prominent lack of IgGl-Fc fucosylation of platelet alloantibodies in pregnancy. Blood 123, 471-481 (2014) for all samples except for the MeV and MuV cohorts that were measured on a Maxis Impact HD quadrupole-time-of-flight MS (Bruker Daltonics, Bremen, Germany). (Glyco-)peptides were trapped with 100% buffer A (0.1% formic acid in water) and separated on a 15 min 0-25% buffer B (95% acetonitrile, 5% water) linear gradient. In the current study we focused on IgG1, without analyzing IgG3 due to its possible interference with IgG2 and IgG4 at the glycopeptide level (H. K. Einarsdottir, et al., Comparison of the Fc glycosylation of fetal and maternal immunoglobulin G. Glycoconj. J. 30, 147-157 (2013)). Mass spectrometry results were extracted and evaluated using FlexAnalysis software (Bruker Daltonics) for all samples except for the MeV, MuV, and malaria cohorts that were analysed with Skyline software. Data was judged reliable when the sum of the signal intensities of all glycopeptide species (Table S1) was at least higher than background plus 10 times its standard division, otherwise the data was excluded (R. Kapur, et al., A prominent lack of IgG1-Fc fucosylation of platelet alloantibodies in pregnancy. Blood 123, 471-481 (2014s). The total level of glycan traits was calculated as described in Table S2.

### Statistical analysis

Statistical analyses were performed using GraphPad Prism version 7.02 for Windows (GraphPad Software Inc., La Jolla, CA, www.graphpad.com). To analyze whether Fc-fucosylation for total and antigen specific IgG differs between the tested cohorts, statistical analysis was performed using t tests. To investigate whether Fc-fucosylation profiles of two specific antibodies in the same individual are correlated statistical analysis was performed using a Pearson correlation. The level of significance was set at P<0.05.

### Results

Immune responses characterized by afucosylated IgG have so far only been observed against foreign proteins found in the cell membrane of the host. Therefore, we hypothesized that immune responses against alloantigens expressed by blood cells mimic programmed immune responses against for example outer-membrane antigens from enveloped viruses, triggering a potent non-fucosylated IgG response (Fig. 1). Unlike soluble-protein antigens and antigens provided by non-enveloped viruses, alloantigens and membrane proteins expressed by enveloped-viruses are expressed on host cells and derived membranes.

Figure 1 shows a hypothetical model: In case of immune response to soluble protein antigen B-cell receptor (BCR, a membrane immunoglobulin) is activated, resulting in the production of normal fucosylated antibodies (Fig. 1A). In case of immune response to allo-antigens, paternal allo-antigen on a Red Blood Cell (RBC) recognized by the BCR, and possibly by a yet unknown immune regulatory receptor-ligand pair providing a signal for self (Fig. 1B). In case of immune response to enveloped viral infections (Fig. 1C), recognition of enveloped-virus infected cells by B cells is similar as for cellular-alloantigen recognition. The initial recognition can potentially occur towards enveloped-virally infected cells and possibly after viral assembly. The proposed signaling in immune response to allo-antigens and immune response to enveloped viral infection causes altered glyco-programming of the B cells culminating in a unique IgG-response characterized by a low fucosylation and enhanced ADCC. The model potentially explains both why immune response to soluble proteins, non-enveloped viruses and cellular pathogens such as bacteria is different to responses to enveloped-viruses and why immune responses to allo-antigens mimic that of an enveloped viral infection.

We therefore analyzed IgG glycosylation, with particular focus on fucosylation, in immune responses in natural infections by enveloped viruses, namely HIV, cytomegolovirus (CMV), Measles virus (MeV), Mumps virus (MuV) and Hepatitis B (HBV). In addition, we compared the Fc-fucosylation patterns of Ag-specific antibodies induced upon natural infection of a non-enveloped virus (Parvovirus B19), vaccination with a protein subunit (HBV vaccine), and live attenuated enveloped viruses (LAV) (MeV and MuV vaccine), to test if the antigen context is indeed important.

To investigate the Fc-glycosylation of total- and antigen-specific antibodies, first IgG from human serum samples was affinity-purified using protein G affinity beads and immobilized antigens, respectively. Thereafter, isolated IgG was digested with trypsin and resulting IgG1-Fc-glycopeptidesanalyzed with liquid chromatography-mass spectrometry (LC-MS) (Fig. 2A) (11, 16, 18). Subsequently, intensities were extracted and IgG-glycosylation profiles were calculated (Fig. 1B-C).

In agreement with our hypothesis, antigen-specific antibodies against the alloantigen human platelet antigen (HPA)-1a showed a strong decrease in fucosylation (Fig. 3A), similar as we have shown previously for other alloantigens (Kapur R, et al. (2014) Blood 123(4):471-480; Sonneveld ME, et al. (2017) Br J Haematol 176(4):651-660).

Analogous to the alloantigens, the response to these enveloped viruses also showed significant afucosylation of the antigen-specific IgG (Fig.3B), but was absent against the non-enveloped virus Parvo B19 (Fig. 3C) and in total IgG (Fig.3A-C). The extent of the response to the enveloped viruses was highly variable, both between individuals and between the types of antigen, which is in agreement with the variable tendency of different RBC-alloantigens to induce an afucosylated response (Sonneveld ME, et al. (2017) Br J Haematol 176(4):651-660). The reduction in fucosylation was particularly strong for CMV and to a lesser degree for HIV (Fig. 3B). The HIV response is in line with what was previously described by Ackerman et al., showing a decreased fucosylation of HIV specific antibodies compared to total IgG (Ackerman ME, et al. (2013) J Clin Invest 123(5):2183-2192).

To test whether some individuals had a greater intrinsic capacity to generate an afucosylated IgG-response than others, we compared IgG-Fc fucosylation levels formed against different antigens within the same individual. No correlation in IgG1 Fc fucosylation was observed between anti-HPAla and anti-CMV in the same individual (Fig. 4a) or between anti-HIV and anti-CMV antibodies in the same individual (Fig. 4b), suggesting that the level of afucosylation is not determined by a general host factor such as genetics but is rather stochastic or multifactorial.

To further investigate the immunological context by which potent low fucosylated IgG is formed, we compared immune responses to identical viral antigens in different contexts. First we compared Hepatitis B surface antigen (HBsAg)-specific antibody glycosylation in humans naturally infected with HBV or vaccinated with the recombinant HBsAg protein (Fig. 5A). Whereas total-IgGl fucosylation levels were similar between the two groups, anti-HBsAg IgG1 fucosylation was elevated in individuals vaccinated with the HBsAg protein both compared to the fucosylation of total IgG of both groups and ag-specific IgG of the naturally infected group . The finding that HBsAg-specific antibodies in individuals that cleared a natural infection showed lowered Fc-fucosylation compared to protein subunit vaccination strongly suggests that a specific context for the antigenic stimulus is required for afucosylated-IgG responses.

We then compared IgG responses to MeV and MuV formed after a natural infection or vaccination with live attenuated viruses (LAV). Unlike the HBV protein subunit vaccine, MeV and MuV LAV vaccines showed a similar Ag-specific Fc-fucosylation compared to their natural infection counterpart (Fig. 5B). For MeV, this was only significantly lowered compared to total IgG-fucosylation in the younger vaccinated cohort (mean age 19.5, Figure 5b right part). The lack of significance between total IgG and antigen specific measles IgG in the naturally infected individuals is likely to be masked by the natural tendency of lowered total IgG-fucosylation with increasing age (de Haan N, et al. (2016) J Proteome Res 15(6): 1853-61), as the naturally infected cohort (before introduction of the MeV/MuV vaccination program in 1980s in the Netherlands) is older than the vaccine cohort (average 63.5 vs 19.5 years, respectively). In line with this, the total IgG fucosylation of the older cohort showed significantly lowered total-IgG fucosylation compared to the younger vaccinated cohort (p<0.01). Similarly, Together, these findings provide solid evidence that antibody fucosylation can be determined by the context in which the antigen is displayed to the immune system. In addition, this suggests that immune response can be tailored by providing a vaccine in the right context, e.g. attenuated viruses. This might also apply to other infectious agents that also replicate within cells and expresses pathogenic proteins on host cells, e.g. *P. falciparum.* One such class of proteins are the *Plasmodium falciparum* erythrocyte membrane protein-1 (PfEMP1)expressed on the surface of infected RBC. Naturally acquired antibodies to these proteins are known to be protective. Regardless of this immunity, which is usually acquired during the first five years of life in an endemic area pregnant women are susceptible to disease, due to a pregnancy-restricted PfEMP1 variant (Hviid L, Salanti A (2007) Parasitology, pp 1871-1876). The fucosylation level of the protective IgG has so far not been studied, but could prove to be of the outmost importance. Especially considering our data from the differentially vaccinated groups and that current malaria vaccine candidates are recombinant proteins (Hviid L (2010) Hum Vaccin 6(1). doi:10.4161/hv.6.1.9602). We therefore investigated if anti-PfEMP1 IgG shows signs of IgG-afucosylation after natural immune response. We selected two PfEMP1 proteins expressed on infected RBC. The VAR6 expressed by parasites in general malaria cases and VAR2CSA expressed solely by parasites in pregnancy-associated malaria (Hviid L, Salanti A (2007) Parasitology, pp 1871-1876). In addition we included the glutamine-rich protein (GLURP) antigen expressed almost exclusively on the extracellular stage of the parasite(merozoites). In accordance with our hypothesis, the immune responses to both PfEMP1 antigens showed a very strong tendency toward afucosylated IgG (Fig. 6A), while GLURP-specific IgG showed almost exclusively fucosylated responses. The level of fucosylation steadily dropped depending on the number of potential exposures (Pregnancies) for the VAR2CSA-specific, while the fucosylation levels were stable and very low for the VAR6-specific antibodies in the adult cohort.

In conclusion, this study supports our hypothesis that antigens residing in host membranes, such as surface membrane proteins in enveloped viruses, intracellular parasites or allogenic transplants, can specifically trigger an afucosylated antibody response. In this context the membrane of (infected) endogenous cells express both exogenous and endogenous antigens providing a platform for co-recognition by host receptors on immune cells that can theoretically sense differential antigen contexts. We hypothesize that this platform is sensed by B cells directly by combining at least two signals provided by the B cell receptor and a yet unknown host receptor-ligand pair (Fig. 1). Alternatively, the differential recognition may be more complex and require additional interactions from antigen-presenting cells, T cells and/or cytokines. Importantly, when IgG afucosylation does occur, the final outcome, namely an afucosylated IgG response, results in a potent immune response, honed for destruction of targets cells by FcγRIIIa- expressing NK cells, monocytes and macrophages but also FcγRIIIb-expressing granulocytes.

### Example 2

In support of our hypothesis and importance of fucosylation for anti-malaria responses and future vaccine development, is the fact that current experimental malaria vaccines are recombinant soluble proteins (e.g. VAR-type malaria pathogenic proteins expressed on RBC). Such vaccines are thus not expected to induce antibodies with afucosylated signatures. A study performed in a *Plasmodium* vaccine cohort, with vaccination based on such recombinant proteins, indeed confirmed that isolated antibodies do not show afucosylation. Thus these antibodies bind with low affinity to FcyRIIIa and FcγRIIIb and induce lower ADCC and phagocytosis, compared to afucosylated antibodies.

### Example 3

### Generation of RBC expressing Plasmodium antigen

### Generation of iPSC

Human Somatic cells such as primary fibroblast or peripheral blood mononuclear cells (PBMCs) obtained from a patient or healthy subject can be reprogrammed into iPSCs. The reprogramming of somatic cells into a pluripotent state can be done by any method known in the art such a transfections or viral transduction to express one or more pluripotent markers (e.g OCT4, SOX2, c-MYC, KLF4 Nanog) and plasmodium antigens of choice.

### Differentiation of iPSC into RBC

Human iPSC: are cultured feeder free in E8 medium (Thermo Fisher Scientific), on matrigel (BD-biosciences)-coated plates. Medium is changed every other day and cells are passaged every 4-5 days, using ReLeSR (Stem cell technologies) as described by manufacturers with a split-ratio of 1: 20. iPSC are differentiated as whole colonies derived from single cells. In short, iPSC are made into single cells and 100-150 cells were plated onto 6 cm dishes. Colonies were allowed to expand to 400 pm size upon which media was changed to differentiation media containing 20 ng/ml BMP4, 40 ng/ml VEGF and 50 ng/ml bFGF in Stemline II (sigma-aldrich), media is refreshed after 3 days. At day 6, this medium is replaced by serum- free humanized IMDM-based medium (Cellquin) (37) supplemented with interleukin 3, interleukin 6, SCF, Nplate (TPO agonist), VEGF, BMP4 and 1 Erythropoietin. Cells were refreshed every 2 days and CD34+ erythroid progenitors harvested between day 9 and 15 were cultured as described before in van den Akker et al. Haematologica, 95, 1594-1598).

### Generation of RBC from PBMC

Human PBMC from whole blood are purified by density separation using Ficoll-Paque (per manufacturer's protocol). PBMC are seeded at 5 to 10 × 10⁶ cells/mL in Cellquin medium based on HEMA-Def7 supplemented with EPO, human recombinant stem cell factor, dexamethasone, and human ultra-clean albumin, referred to as expansion medium (EM). Interleukin-3 is added to EM on the first day (stage 1). Media was partially replenished every 2 days with EM. Around day 6, upon erythroblast detection, the cells are maintained 1 to 2 × 10⁶ cells/mL for 15 to 25 days (stage 2). Plasmodium antigens can be introduces by standard transfection or retroviral transduction at this stage or already at the PBMC stage as described above. Erythroblasts differentiation (stage 3) is induced in differentiation medium (DM) containing Cellquin supplemented with EPO, Omniplasma (Octopharma, Wien, Austria), holotransferrin (Sanquin, The Netherlands), and heparin. At day 2, half of a medium change is performed. At day 5, storage components (Sanquin Plasma Products) are added and media is refreshed (half) every 2 days until fully differentiated at days 10 through 12 of differentiation. (Heshusius et al. Blood Adv. 2019 Nov 12; 3(21): 3337-3350).

## Claims

1. A mammalian cell or a cell membrane containing fraction thereof comprising a viral, bacterial, parasitic or fungal antigen at its cell surface.

2. A cell or fraction thereof according to claim 1, wherein the cell is selected from the group consisting of a red blood cell or a precursor thereof and a thrombocyte or a precursor thereof.

3. A cell or fraction thereof according to claim 1, wherein the antigen is a *Plasmodium* antigen.

4. A cell or fraction thereof according to claim 1, wherein the cell is a red blood cell and comprises a *Plasmodium* antigen at its cell surface.

5. A cell or fraction thereof according to any one of the preceding claims wherein said cell or fraction thereof expresses said antigen at the cell surface.

6. A live attenuated enveloped virus comprising a *Plasmodium* antigen or a nucleic acid sequence encoding said *Plasmodium* antigen or comprising a tumor-associated antigen or a nucleic acid sequence encoding said antigen.

7. A cell or fraction thereof according to any one of claims 3-5, or a live attenuated enveloped virus according to claim 6, wherein said antigen is selected from the group consisting of *P. falciparum* erythrocyte membrane protein 1 (PfEMP1), pregnancy associated functional and/or structural homologs of PfEMP1 such as VSAPAM (VAR2CSA) or functional homologues thereof, malaria surface targets on various stages of development including *P. falciparum* Schizont Egress Antigen-1 (PfSEA-1), *P. falciparum* reticulocyte-binding protein homologue 5 (PfRH5), RIFIN, STEVOR, SURFIN, GLURP, Duffy binding protein (DBP), liver stage antigen 1 (LSA1), Merozoite surface protein 1 (MSP1), *P. falciparum* sporozoite surface protein 2 (PfSSP2), Circum-Sporozoite Protein (CSP), serine repeat antigen and AMA-1, PfMC-2TM, VIR, KIR and SICAvar.

8. A live attenuated virus according to claim 6 or 7, wherein the virus is selected from the group consisting of herpes simplex virus, varicella-zoster virus, poxyvirus (e.g. Modified Vaccinia Ankara), cytomegalovirus, Epstein-Barr virus, vaccinia virus, Dengue virus, hepatitis virus, yellow fever virus, influenza virus, mumps virus, respiratory syncytial virus, retrovirus and coronavirus.

9. A cell or fraction thereof or a live attenuated enveloped virus according to any one of the preceding claims, wherein said cell or fraction thereof, or said virus is capable of stimulating an afucosylated IgG response in a subject.

10. A pharmaceutical composition comprising a cell or fraction thereof or a live attenuated enveloped virus according to any one of claims 1-9 and a pharmaceutically acceptable carrier, diluent and/or excipient.

11. A vaccine comprising a cell or fraction thereof or a live attenuated enveloped virus according to any one of claims 1-9 and a pharmaceutically acceptable carrier, diluent and/or excipient.

12. A cell or fraction thereof or a live attenuated enveloped virus according to any one of claims 1-9 for use as a vaccine.

13. A cell or fraction thereof or a live attenuated enveloped virus according to any one of claims 1-9 for use in the treatment or prevention of a viral, bacterial, parasitic or fungal infection.

14. A cell or fraction thereof or a live attenuated enveloped virus according to any of claims 1-9 for use in the treatment or prevention of malaria.

15. A live attenuated enveloped virus according to claim 6 comprising a tumor-associated antigen or a nucleic acid sequence encoding said antigen for use in the treatment of cancer.

16. A cell or fraction thereof or a live attenuated enveloped virus according to any of any one of claims 1-9 for use in inducing and/or stimulating an afucosylated IgG response against an antigen of interest in a subject, wherein said cell or fraction thereof comprises said antigen of interest at its cell surface, or said virus comprises said antigen of interest or a nucleic acid encoding said antigen of interest, preferably wherein said afucosylated IgG molecules have increased binding to the FcyRIIIa and/or FcyRIIIb receptor.

17. A cell or fraction thereof or a live attenuated enveloped virus according to any of any one of claims 1-9 for use in inducing an immunogenic response against an antigen of interest in a subject, wherein said cell or fraction thereof comprises said antigen of interest at its cell surface, or said virus comprises said antigen of interest or a nucleic acid encoding said antigen of interest.
